# EUROPEAN PATENT SPECIFICATION

(11) **EP 2 035 442 B1**
(45) Date of publication and mention of the grant of the patent: **12.10.2011**
(21) Application number: 07765429.1
(22) Date of filing: 14.06.2007
(51) Int. Cl.: C07K 1/113, C07K 7/06

(54) **NEW SUPPORTED OXIDATION REACTANTS, PROCESS FOR THEIR PREPARATION AND USES THEREOF**
NEUE GETRÄGERTE OXIDATIONSREAKTANTEN, VERFAHREN ZU DEREN HERSTELLUNG UND DEREN ANWENDUNGEN
NOUVEAUX RÉACTIFS D'OXYDATION SUPPORTÉS, LEURS PROCÉDÉS DE SYNTHÈSE ET LEURS APPLICATIONS

(30) Priority: 14.06.2006 US 813371 P
(43) Date of publication of application: 18.03.2009
(73) Proprietor: CENTRE NATIONAL DE LA RECHERCHE SCIENTIFIQUE (CNRS), 75016 Paris (FR); Université de Montpellier I, 34006 Montpellier (FR); Genepep, 34830 Clapiers (FR)
(72) Inventor: MARTINEZ, Jean, F-34720 Caux (FR); SUBRA, Gilles, F-34990 Juvignac (FR); CRISTAU, Michèle, F-34130 St Aunes (FR); CANTEL, Sonia, F-06100 Nice (FR)
(74) Representative: Warcoin, Jacques
(86) International application number: PCT/EP2007/055917
(87) International publication number: WO 2007/144411

(56) References cited:
- US-A- 5 777 076
- ANONYMOUS: "Product No. HCBB16" PRODUCT CATALOG HECHENG S&T, [Online] 2 March 2005 (2005-03-02), XP002448998 Retrieved from the Internet: URL:http://web.archive.org/web/20050302213 632/http://www.tjhecheng.com/en/MBHA-Resin s.htm> [retrieved on 2007-09-07]
- "Fluka Catalog 2005/2006" 2005, SIGMA-ALDRICH , XP002449011 Page 1480; Compound Boc-Met(O)-PAM resin; Cat. No. 18752
- FERRER T ET AL: "Application of the disulfide trapping approach to explain the antiparallel assembly of dimeric rabbit uteroglobin: A preliminary study using short peptide models" LETTERS IN PEPTIDE SCIENCE 1999 NETHERLANDS, vol. 6, no. 2-3, 1999, pages 165-172, XP002449009 ISSN: 0929-5666
- SNOW J T ET AL: "Oxidation of sulfhydryl groups to disulfides by sulfoxides." BIOCHEMICAL AND BIOPHYSICAL RESEARCH COMMUNICATIONS 5 MAY 1975, vol. 64, no. 1, 5 May 1975 (1975-05-05), pages 441-447, XP008082991 ISSN: 0006-291X
- LIU Y ET AL: "Modification of the Swern Oxidation: Use of Stoechiomnetric Amounts of an Easily Separable, Recyclable, and Odorless Sulfoxide That Can Be Polymer-Bound" JOURNAL OF ORGANIC CHEMISTRY, AMERICAN CHEMICAL SOCIETY. EASTON, US, vol. 61, no. 22, 1996, pages 7856-7859, XP002215342 ISSN: 0022-3263
- CHOI M K W ET AL: "Soluble polystyrene-based sulfoxide reagents for Swern oxidation reactions" TETRAHEDRON, ELSEVIER SCIENCE PUBLISHERS, AMSTERDAM, NL, vol. 59, no. 36, 1 September 2003 (2003-09-01), pages 7171-7176, XP004448528 ISSN: 0040-4020
- GAIT R J ET AL: "USE OF N CARBOXY ANHYDRIDES IN THE SYNTHESIS OF DI PEPTIDES THE IMPROVEMENT OF ISOLATED YIELDS BY THE FACILE REMOVAL OF BUFFER COMPONENTS" CANADIAN JOURNAL OF CHEMISTRY, vol. 50, no. 2, 1972, pages 299-303, XP008083139 ISSN: 0008-4042

## Description

The present invention relates to novel supported oxidation reactants, to processes for preparing them, and to their use for the formation of intramolecular disulfide bridges.

The use of supported reactants is fast-expanding in organic chemistry, in particular since the automation of organic synthesis. Supported reactants are of very great use since, after the reaction, they can be eliminated from the reaction medium by simple filtration. Some can even be recycled and reused. In this case, it is advantageous to note that these supported reactants fall nicely within the approach of the long-lasting development and respect of the environment.

Supported reactants are particularly advantageous for promoting intramolecular reactions. In fact, they are known to cause a phenomenon of "pseudodilution" which makes it possible to minimize polymerizations and to use much smaller amounts of solvents.

The synthesis of peptides is responsible for supported synthesis. It is today highly automated and the elongation of the peptide chain is generally carried out on a solid support. However, in certain cases, the final synthesis and treatment steps are carried out in solution, after detachment of the peptide chain from the solid support. This is in particular the case of the formation of disulfide bridges.

The great biological advantage of disulfide bridges in the peptide field should be noted. The biological activity of a peptide is linked to its primary structure, i.e. to the series of amino acids, and also to its three-dimensional structure. The latter is conditioned by the presence, within the same peptide chain, of varied interactions such as hydrogen bonds or hydrophobic interactions, and also by the existence of covalent bonds such as those involved in disulfide bridges. The presence of these disulfide bridges will make it possible for the peptide to structure itself in space and to adopt a conformation which allows it to acquire a biological activity essential to its participation in varied physiological processes.

Usually, the intramolecular formation of disulfide bridges requires conditions of high dilution and the presence of an oxidant. Snow et al, Biochem Biophys Res Comm, 1975, 64(1), 441, disclose oxidation of -SH groups to disulfides by sulfoxides. Dimethyl sulfoxide (DMSO) is one of the oxidizing reactants most commonly used for his. This product has the advantage of being water-soluble. One of its major drawbacks is its elimination from the reaction medium, which requires evaporation under strong vacuum or repeated lyophilizations. Furthermore, the dimethyl sulfide generated during the reaction is volatile and toxic.

The use of supported oxidation reactants has subsequently been envisaged for the conventional synthesis of bioactive peptides, but also for the automated synthesis of chemical libraries of peptides bearing disulfide bridges. They exhibit numerous advantages:
- since the formation of disulfide bridges is an intramolecular reaction, these supported reactants avoid having to work under very dilute conditions, minimizing the volume of solvent and allowing miniaturization and the use of robot wells with a volume which is often small;
- the supported reactants make it possible to obtain the bridges in the peptides and the proteins under very mild and simple conditions without the use of toxic, foul-smelling or volatile oxidants;
- the supported oxidation reactants can be eliminated simply and do not require any lyophilization or evaporation, which greatly facilitates automation;
- the supported oxidation reactants can be recycled and reoxidized after use.

Few supported oxidation reactants which are effective for the formation of disulfide bridges have been described to date.
6-(Methylsulfinyl)hexanoic acid, fixed on a solid support of polyethylene glycol-polystyrene (PEG-PS) type, is a good DMSO substitute for the synthesis ofketones by Swem oxidation reaction, but has not been used for the formation of disulfide bridges (for example: Liu, Vederas, J. Org. Chem., 1996, 61, 7856; Choi, Toy, Tetrahedron, 2003, 59, 7171). Only the Ellman reagent (5,5'-dithiobis(2-nitrobenzoic) acid or DTNB), developed initially for measuring the concentration of free thiols, has been adapted for the formation of disulfide bridges, as a supported reactant (J. Peptide Res 63, 2004, p. 303-312). It remains, however, expensive to manufacture and relatively ineffective compared with a conventional oxidation method in solution using DMSO.

There remains therefore a need for novel supported oxidation reactants, which are at least as effective as the reactants used in solution, are easy to use, and are inexpensive.

The applicant has developed the synthesis and the use of novel oxidation reactants on a solid or soluble support, capable of promoting the formation of disulfide bridges, in particular in peptides. These reactants can be used for the conventional synthesis of peptides with disulfide bridges, but also for the parallel synthesis of peptide libraries (for example, in 96-well or 384-well plates). These reactants are easy to use and produce oxidation products under much better conditions than the existing reactants. Furthermore, their cost, and the ease with which they are synthesized and used makes them choice reactants for the formation of disulfide bridges in peptides. These reactants consist of a series of oxidized methionines grafted onto a solid or liquid support.

The present invention therefore relates to an oxidation reactant of formula (I): 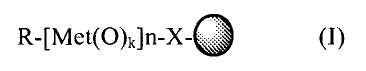 in which,
■ R represents a hydrogen atom or an amine function-protecting group,
■ Met represents a methionine residue of formula 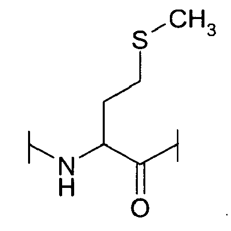
■ [Met(O)ₖ] represents an oxidized methionine residue of formula 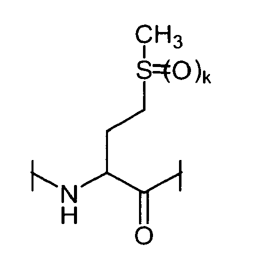
■ 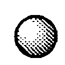 represents a solid or soluble support used in organic synthesis,
■ X represents a radical derived from a function of the type amine or hydroxyl, or halogeno, capable of anchoring methionine, it being understood that this function capable of anchoring methionine can be separated from the support by means of a functionalized arm or a spacer, and
■ n is between 2 and less than 10000, and
■ k is 1 or 2, or
■ n is equal to l and k is equal to 2.

For the purpose of the present invention, the term "amine function-protecting group" is intended to mean any substituent which protects the amine group of the peptide chain against unwanted reactions. The various groups that come under this definition are widely known and described, for example, in "Protective Groups In Organic Synthesis", John Wiley & Sons, New York, 1981. In a nonlimiting manner, mention may be made of protection of the amine function in the form of carbamates, of amides, of N-alkylated derivatives, of aminoacetals, of N-benzyl derivatives, of imines or of enamines. Thus, among the protective groups, mention may be made of linear or branched (C₁-C₆) alkylcarbonyl, linear or branched (C₁-C₆) alkoxycarbonyl, linear or branched (C₂-C₆) alkenyloxycarbonyl, linear or branched (C₁-C₆) alkynyloxycarbonyl, aryloxycarbonyl, linear or branched (C₁-C₆) arylalkyl, arylcarbonyl, arylsulfonyl, arylaminocarbonyl, or linear or branched (C₁-C₆) alkylaminocarbonyl groups, it being possible for each of these groups to be substituted with one or more halogen atoms or hydroxyl, amino, or linear or branched (C₁-C₆) mono- or dialkylamino groups. By way of example, mention may be made of acetyl, benzoyl, benzyl, phenylsulfonyl, tert-butyloxycarbonyl (BOC), benzyloxycarbonyl (Z), para-methoxybenzyloxycarbonyl, para-nitrobenzyloxycarbonyl, trichloroethoxycarbonyl (TROC), allyloxycarbonyl (Alloc), 9-fluoromethyloxycarbonyl (Fmoc), trifluoroacetyl, and benzyl carbamate groups.

In a preferred aspect of the invention, when it represents an amine function-protecting group, the R group will be chosen from linear or branched (C₁-C₆) alkylcarbonyl, linear or branched (C₁-C₆) alkyloxycarbonyl, linear or branched (C₁-C₆) alkenyloxycarbonyl, linear or branched (C₁-C₆) alkynyloxycarbonyl, and aryloxycarbonyl groups, it being possible for each of these groups to be substituted with one or more halogen atoms or hydroxyl, amino, or linear or branched (C₁-C₆) mono- or dialkylamino groups. Mention may, for example, be made of acyl, acetyl, benzyloxycarbonyl (Z), tert-butyloxycarbonyl (BOC), or 9-fluorenylmethyloxycarbonyl (Fmoc) groups.

The solid or soluble supports suitable for the implementation of the present invention are chosen from supports conventionally used in organic chemistry, and particularly in peptide synthesis. These supports used in the context of the present invention are functionalized with a function capable of anchoring the oxidized methionine. In this respect, mention may be made of amine or alcohol functions, or else substitutable halogen atoms.

For the purpose of the present invention, the term "radical derived from a function of the type amine, or hydroxyl, or halogeno" is intended to mean a radical resulting from the attachment of the methionine residue to a solid support functionalized with an amino function, a hydroxyl function or a substitutable halogeno function.

In an advantageous aspect of the invention, X represents a radical NH.

In another aspect of the invention, X represents an oxygen atom.

In another advantageous aspect of the invention, the support 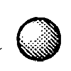 is chosen from gelatinous or macroporous supports having a matrix with a polystyrene, polyamide, polyethylene glycol or composite base, it being possible for said supports to be in the form of beads, of film-coated supports such as rings or lanterns, of plugs, or of noncrosslinked soluble supports, and it being possible for said matrix to be nonfunctionalized or functionalized with a functionalized arm.

For the purpose of the invention, the term "functionalized arm or spacer" is intended to mean any fragment commonly grafted to the solid support as defined above, in particular those used for peptide synthesis.

As support suitable for the implementation of the invention, mention may be made more particularly of resins without a functionalized arm or functionalized resins such as chloromethyl polystyrene, benzyloxybenzyl alcohol polystyrene, aminomethyl polystyrene or methylbenzhydrylamine polystyrene resins, or resins grafted with a functionalized arm chosen from Rink amide (Rink signifying 4-[2',4'-dimethoxyphenyl-(9-fluoromethyloxycarbonyl)aminomethyl]phenoxy-), chlorotrityl, hydroxymethylbenzylacetamide, Sieber amide (Sieber signifying 9-aminoxanthen-3-yloxy-), aminomethyl-3,5-dimethoxyphenoxyalkyl, aminomethyl-3-dimethoxyphenoxyalkyl, hydroxymethyl-3,5-dimethoxyphenoxyalkyl, and hydroxymethyl-3-dimethoxyphenoxyalkyl.

The types of solid supports that are preferred according to the invention are:
- (i) either of the type beads of gelatinous or macroporous resins having a matrix with a polystyrene base, such as aminomethyl polystyrene or 4-methylbenzhydrylamine polystyrene, or having a matrix with a polyamide (PL) or polyethylene glycol base (PEG), or else composite supports of polyethylene glycol-polystyrene (PEG-PS) or polyethylene glycol-dimethylacrylamide (PEGA) type,
- (ii) or of film-coated type such as SynPhase Lanterns^{®} (Mimotopes).

The preferred soluble supports used for grafting the oxidized methionines are of polyethylene glycol (PEG) type.

The oxidation reactant according to the present invention comprises several [Met(O)] units or [Met(O)₂] units. The number n thereof will be less than 10 000, and preferably less than 1000. In an advantageous aspect of the invention, n is less than 100, and more particularly less than 50. In another advantageous aspect of the invention, this number n is between 2 and 15.

As indicated above, the oxidation reactants according to the present invention are intended to induce the formation of S-S covalent bonds between two sites of the same molecule. The formation of these intramolecular disulfide bridges will in particular have the aim of giving a specific spatial conformation to said molecule.

Thus, the invention relates to the use of an oxidation reactant of formula (I) as defined above, for the formation of intramolecular disulfide bridges. More advantageously, it relates to the use of an oxidation reactant of formula (I) according to the invention, for the formation of intramolecular disulfide bridges in peptides and/or proteins. Finally, a particularly advantageous aspect of the invention relates to the use of an oxidation reactant of formula (I) as defined above, for the preparation of a library of peptides comprising at least one intramolecular disulfide bridge, by high-throughput parallel synthesis.

By multiplying the number of compounds synthesized simultaneously, automated or manual parallel synthesis techniques make it possible to rapidly generate vast collections of compounds. These techniques have been of great benefit to the search for novel biologically active compounds by allowing the structural modulation of lead compounds, but are also used for the optimization of reaction conditions. Miniaturization and robotization have contributed to the success of parallel synthesis, but one of the keys to its effectiveness lies in the simplification of the tasks in order to facilitate the automation thereof. Thus, the use of supported reactants or of resins which avoid the delicate purification steps makes it possible, when this is possible, to increase the productivity and the rapidity of parallel syntheses.

The present invention also relates to the process for preparing the oxidation reactants of formula (I). Several synthetic pathways have been developed. Those skilled in the art will choose the synthesis that is most suitable, according to the exact nature of the oxidation reactant that they wish to obtain, to the availability of the starting raw materials and to the supports used.

A first process for preparing an oxidation reactant of formula (I) is characterized in that the compound of formula (II): 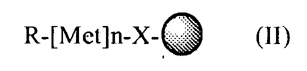 in which R, n and X are as defined above, and 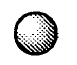 is subjected to an oxidation reaction, to give the compound of formula (I): 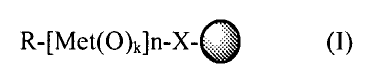 in which R, n, k, X and 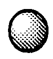 are as defined above,

The oxidation reaction can be carried out using any mild oxidation reactant conventionally used to obtain a sulfoxyde derivative (k=1), and in an advantageous aspect, will be carried out with aqueous hydrogen peroxide.

The oxidation reaction can be carried out using any stronger oxidation reactant to obtain a sulfone derivative (k=2), and in an advantageous aspect, will be carried out with MCPBA (meta chloro perbenzoic acid).

The compound of formula (II) is prepared in two different ways. The first synthetic pathway uses, as substrate, the compound of formula (III):

R-[Met]-OH (III)

in which R is as defined above,
which,
by reaction with a support of formula 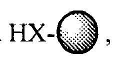 in which X and 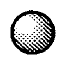 have the same meaning as above,
gives the compound of formula (IV): 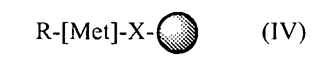 in which R, X and 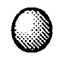 are as defined above, which compound of formula (IV), after deprotection by cleavage of the R group, is subjected to a series of reactions with a compound of formula (III):

R-[Met]-OH (III)

in which R is as defined above,
the reaction being repeated n-1 times to give a compound of formula (II): 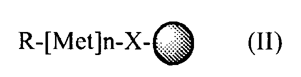 in which R, n, X and 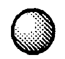 are as defined above,
it being understood that the protective group R can be removed and/or modified at any moment of the synthesis depending on the reactants used.

In the above process, the compound of formula (III), the N-terminal portion R of which is in the protected form, for example protected with an Fmoc group, is grafted onto the solid support using a peptide coupling reaction involving a coupling agent (for example, 1-H-benzotriazol-1-yloxytris(dimethylamino)phosphonium hexafluorophosphate (BOP)) in a basic medium. The amine function-protecting group is then removed so as to be able to again use the deprotected derivative (IV) in a coupling reaction with the same compound of formula (III) protected on its N-terminal portion in the form of Fmoc.

The second method for preparing the compound of formula (II) above consists in reacting the N-carboxyanhydride of formula (V): 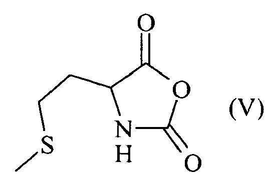 by polymerization in the presence of a support of formula 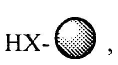 in which X and 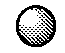 have the same meaning as above, in a solvent such as THF, for a period of
between 12 and 48 hours,
to give a compound of formula (II'): 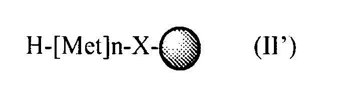 an analog of the compound of formula (II) in which R represents a hydrogen atom, it being possible for the latter to be replaced with a protective group, to give a compound of formula (II) as defined above.

In this alternative to the method for preparing the compound of formula (II), the compound of formula (V) is reacted with a functionalized resin, for example functionalized with a primary amine, to give the polymer of formula (II). The length of this polymer, i.e. the value of the number n, depends on the concentration of compound of formula (V) relative to the primary amine load of the resin, and on the operating conditions such as the duration and the temperature of the reaction, or the solvent used.

A second process for preparing an oxidation reactant of formula (I) is characterized in that the compound of formula (VI):

R-[Met(O)ₖ]-OH (VI)

in which R and k are as defined above, and k is 1 or 2
is condensed with a support of formula 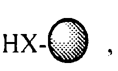 in which X and 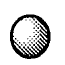 have the same meaning as above,
to give a compound of formula (VII): 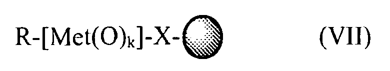 in which R, k, X and 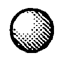 are as defined above,
which compound of formula (VII), after deprotection by cleavage of the R group, is subjected to a series of reactions with a compound of formula (VI):

R-[Met(O)ₖ]-OH (VI)

in which R and k are as defined above,
by means of a conventional peptide coupling method, in the presence of a coupling agent, the reaction being repeated n-1 times, to give a compound of formula (I) as defined above.

This second process for preparing the compound of formula (I) is similar to the first process described above, and uses, as substrate, an already oxidized methionine derivative of formula (VI), which is grafted by peptide coupling onto a support functionalized with an amine function under conditions similar to those mentioned above for the first production process.

A third process for preparing an oxidation reactant of formula (I) is characterized in that the compound of formula (IX): 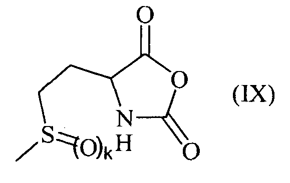 in which k is as defined above,
is polymerized in the presence of a support of formula 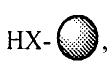 in which X and 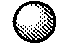 have the same meaning as above, in a solvent such as THF, for a period of between 12 and 48 hours,
to give a compound of formula (I'): 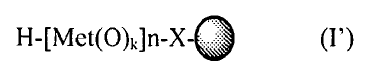 in which n, k, X and 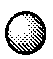 have the same meaning as above,
the N-terminal portion of which compound (I') is protected with an R group, to give a compound of formula (I) as defined above.

The operating conditions of this third production process are similar to those mentioned above for the compound of formula (V), the only difference being that the substrate of formula (IX) is derived from oxidized methionine.

A fourth process for preparing an oxidation reactant of formula (I) is characterized in that the compound of formula (X):

R-[Met(O)ₖ]ₙ-OH (X)

in which R, n and k are as defined above,
reacts with a support of formula 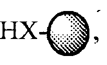 in which X and 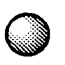 have the same
meaning as above,
to give the compound of formula (I) as defined above.

The compound of formula (X) as defined above can be obtained according to a first synthesis scheme, by means of a series of reactions consisting of condensation of the compound of formula (III'):

H-[Met]-OR' (III')

in which R' represents a linear or branched (C₁-C₆) alkyl, linear or branched (C₂-C₆) alkenyl, benzyl, carboxamidomethyl, or benzhydryl glycol amide ester group,
with a compound of formula (III):

R-[Met]-OH (III)

in which R is as defined above,
by means of a conventional peptide coupling method, in the presence of a coupling agent, the reaction being repeated n-1 times, to give, after protection of the C-terminal function, a compound of formula (XI):

R-[Met]n-OR' (XI)

in which R, R' and n are as defined above, it being understood that R is different from a hydrogen atom,
which compound of formula (XI) is subjected to an oxidation reaction, followed by a deprotection reaction by cleavage of the R' group, to give a compound of formula (X) as defined above.

The above process for preparing the compound of formula (X) makes use of the conventional technologies of peptide synthesis in solution. The N-terminal (R group) and C-terminal (R' group) portions are alternatively in the protected or non-protected form, so as to allow a polycondensation to give a peptide compound of formula (XI), which is then oxidized by means of a suitable method, for example with aqueous hydrogen peroxide, to give the compound (X).

The compound of formula (XI) mentioned above can also be prepared from the N-carboxyanhydride of formula (V): 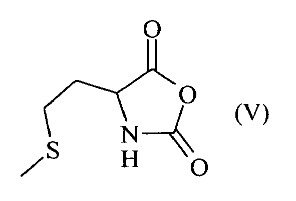 which compound of formula (V) is left in the open air without solvent for 2 days, to give, by means of a spontaneous polymerization reaction initiated by the moisture in the air, the compound of formula (XI'):

H-[Met]n-OH (XI')

in which n is as defined above,
an analog of the compound of formula (XI) for which R and R' each represent a hydrogen atom, it being possible for the latter to be replaced with a protective group, to give a compound of formula (XI) as defined above.

The compound of formula (X) as defined above can also be obtained by means of a second process, by polymerization of the N-carboxyanhydride of formula (IX): 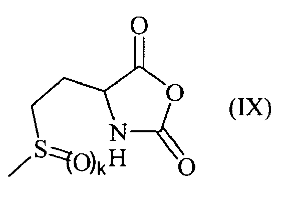 in which k is as defined above,
which is left in the open air without solvent for 2 days, the spontaneous polymerization reaction being initiated by the moisture in the air,
to give a compound of formula (X'):

H-[Met(O)ₖ]n-OH (X')

in which n and k are as defined above,
the N-terminal portion of which is protected with an R group, to give a compound of formula (X) as defined above.

This process for preparing the compound of formula (X) from the N-carboxyanhydride of formula (IX) is carried out under conditions similar to those mentioned above for the preparation of the compound of formula (XI) described above.

The examples which follow serve to illustrate the invention, . The preparation of a supported oxidation reactant is described therein.

Model peptide sequences were then selected from natural peptides having two cysteines and in order to attempt to rapidly cyclize them with the solid-supported reactants of formula (I) according to the invention. The results obtained were compared with the methods described for peptide oxidation using DMSO or the ELLMAN supported reactant. For all these peptides, the supported reactants according to the invention have proved to be much simpler to use, with the possibility of automating the process of formation of the disulfide bonds in parallel. Similarly, they produce better yields and greater purity of the cyclized peptides, without the use of large dilutions usually necessary in order to avoid the formation of polymers.

### Example 1: Preparation of a sulfoxyde type (k=1) supported oxidation reactant on Rink-amide-PS resin:

### Step 1: Protocol for polymerization of methionine N-carboxyanhydride on Rink-Amide-PS resin:

The methionine N-carboxyanhydride (Met-NCA) is solubilized in a minimum amount of anhydrous THF (40 ml for 7 g of anhydride) under argon, the resin is then added (1/100 equivalent relative to Met-NCA) and the entire mixture is stirred for 48 hours.
The resin is then washed with the following solvents: tetrahydrofuran, dimethylformamide, methanol and then dichloromethane.
The Rink Amide-PS resin used has a primary amine function load of 0.98 mmol/g.
On the resin thus functionalized, an aliquot is cleaved using trifluoroacetic acid and analyzed by direct introduction ES+ mass spectrometry and MALDI-Tof mass spectrometry.

### Results obtained:

Direct introduction in the positive electrospray mode into a Micromass Platform II mass spectrometer shows the presence of singly charged ions [M+H]⁺ = 149.0, 280.0, 410.9, 541.9, 672.9, 804.0, 935.0 corresponding to the C-terminal amidated methionine polymers for n ranging between 1 and 7.
MALDI-Tof mass spectrometry analysis using a 2,5-dihydroxybenzoic acid matrix also shows the presence of singly charged ions derived from cationization by sodium and potassium [M+Na]⁺ and [M+K]⁺ for the C-terminal amidated methionine polymers for n ranging between 5 and 9.

### Step 2: Acetylation of the resin

The resin obtained in step 1, bearing methionine polymers, is acetylated for 5 minutes with a 50/50 acetic acid/dichloromethane solution (20 ml for 1.5 g of resin dried in a desiccator) in rotary solid-phase reactors, and then filtered. The acetylation is begun again for 10 minutes. The resin is then washed twice with dichloromethane, twice with methanol, and then twice with dichloromethane.

### Step 3: Mild oxidation of the supported polymethionine to supported polymethionine sulfoxyde (k=1)

The resin obtained in step 2 is then placed in 35 wt% aqueous hydrogen peroxide (20 ml for 1.5 g of resin dried in a desiccator) and left to stir for 1h30 min. The resin is then washed twice with dichloromethane, twice with methanol, and then twice with dichloromethane.

The resin thus obtained can be used directly for forming intramolecular disulfide bonds.

### Example 2: Preparation of a supported oxidation reactant on amino-PEG-PS resin

The supported oxidation reactant on amino-PEG-PS (polyethylene glycol-polystyrene) resin is obtained according to a protocol identical to that described for example 1, replacing the Rink Amide-PS resin with an amino-PEG-PS resin having a primary amine function load of 0.19 mmol/g.

### Example 3: Preparation of a supported oxidation reactant on amino-PEG-PS resin of NovaSyn^{®} type

The supported oxidation reactant on amino-PEG-PS resin of NovaSyn^{®} type is obtained according to a protocol identical to that described for example 1, replacing the Rink Amide-PS resin with an amino-PEG-PS resin of NovaSyn^{®} type having a primary amine function load of 0.3 mmol/g. This resin differs from the resin used in example 2 by virtue of the link between the polyethylene glycol and the polystyrene, which is of the phenylethyl instead of benzyl type.

### Example 4: Preparation of a supported oxidation reactant on PL-PEGA resin

The supported oxidation reactant on PL-PEGA (polyamide-polyethylene glycol copolymer) resin is obtained according to a protocol identical to that described for example 1, replacing the Rink Amide-PS resin with a PL-PEGA resin having a primary amine function load of 0.2 mmol/g.

### Example 5: Cyclization of a peptide by formation of disulfide bridges

The supported oxidation reactant obtained in example 4 was used to form an intramolecular bond between the two cysteines of the following model peptide:

H-Trp-Cys-Ala-Gly-Trp-Cys-Leu-NH₂

The model peptide was solubilized in a 75/25 water/acetonitrile mixture at a concentration of 2.5 mM. 2 ml of this solution (5 µmol) were vortexed (600 rpm) with 50 mg of supported reactant obtained in example 4 (2 equivalents (10 µmol) calculated from the theoretical load of the reactant of example 4). The same experiment was carried out without supported reactant. Samples were taken from each flask at t = 2h45 and t = 50h30. The cyclized oxidized peptide [M+H]+ = 835.35 and the nonoxidized peptide [M+H]+ = 837.35 were detected by LC/MS coupling in the ES+ mode. The percentage of oxidized peptide was estimated by comparing the areas of the chromatographic peaks obtained at 214 nm.
The results are reported in table (I) below:

**Table (I): percentage of oxidized peptide as a function of the oxidation reactant**

| | *t=2h45* | *t=2h45* | *t=50h30* | *t=50h30* |
|---|---|---|---|---|
| Oxidation reactant | Ex 4 | - | Ex 4 | - |
| % oxidized peptide | 91% | 5% | 100% | 11% |

We were able to observe that the supported Poly-Met(O) reactant on PL-PEGA resin was capable of forming the disulfide bridges in the model peptide with a yield of 91 % after 2h45 of reaction at ambient temperature. No other by-product was detected. The yield is quantitative beyond 50 hours.

### Example 6: Cyclization of the CCAP peptide by formation of disulfide bridges:

The formula of the CCAP peptide is as follows:
H-Pro-Phe-Cys-Asn-Ala-Phe-Thr-Gly-Cys-NH₂
Six experiments were carried out in parallel, combining 2 buffers and 3 oxidation reactants.

### - Buffer A, pH = 6.07

Solution of 100 ml of 5% acetic acid to which (NH₄)₃CO₃ is added until the desired pH of 6.3 is attained (approximately 5 g).

### - Buffer B, pH = 7.54

Solution 1: 2.4 g of NaH₂PO₄ in 100 ml of milliQ water
Solution 2: 2.84 g of Na₂HPO₄ in 100 ml of milliQ water
42 ml of solution 2 are added to 8 ml of solution 1, and the volume of the mixture is made up to 100 ml with water in a volumetric flask.

3 oxidation reactants were tested in parallel:
- DMSO in solution at 10% in the chosen buffer,
- the supported Ellman reactant,
- Ac-[Met(O)]₅ supported on NH₂-PEG-PS

The CCAP peptide is solubilized in the buffers at a concentration of 3 mM and aliquots of 2 ml of the solution obtained are distributed into each of the 6 *BOHDAN* "mini-block" reactors in parallel. The three oxidation reactants were used at 5 equivalents relative to the amount of peptide to be oxidized. The amount of supported reactant (5 eq) is calculated relative to the load of the resin.

Thus, 125 mg (5 eq) of Ac-[Met(O)]₅ supported on NH₂-PEG-PS functionalized at 0.24 mmol/g were added to 2ml of CCAP peptide solution. 150 mg (5 eq) of supported Ellman reactant functionalized at 0.20 mmol/g were added to 2 ml of CCAP peptide solution.

The six reactors were shaken on an orbital shaker (600 rpm) at ambient temperature for 45 hours and samples of 40 µl made up to 200 µl with a CH₃CN/H₂O (50/50) solution and then filtered were immediately subjected to LC/MS analyses. The LC/MS experiments were carried out on a quadripolar/time of flight mass spectrometer of Micromass Q-Tof type coupled to a Waters Alliance HPLC system. These analyses made it possible to quantify the presence of cyclized oxidized peptide as a function of time. The percentage of oxidized peptide given in the results table (II) was calculated as a function of the areas of the chromatographic peaks obtained at 214 nm. This value therefore takes into account the possible presence of by-products, but not the presence of DMSO, in samples 3 and 4.

Table (II) Percentage of oxidized peptide as a function of the oxidation reactant.

| **# Exp.** | **Oxidant** | **Buffer** | **15 min** | **4h** | **26 h** |
|---|---|---|---|---|---|
| **1** | Supported Ellman | A | 54% | 89% | 79% |
| **2** | | B | 58% | 69% | 56% |
| **3** | 10% DMSO | A | 13% | 52% | 79% |
| **4** | | B | 15% | 47% | 56% |
| **5** | [Met(O)]₅-PEG-PS | A | 22% | 68% | 100% |
| **6** | | B | 40% | 72% | 86% |

Under the conditions tested, the supported reactant according to the invention bearing 5 oxidized methionines proves to be more rapid than DMSO in solution and less rapid that the supported Ellman reactant. On the other hand, it is the only one which allows the quantitative formation of cyclized peptide in 26 hours without any reaction by-product in a buffer at pH = 6.07.

The chromatograms obtained confirm, in this case, the absence of these by-products, in particular the absence of covalent dimer derived from an intermolecular reaction between two CCAP peptides.

### Example 7: Preparation of a sulfone type (k=2) supported oxidation reactant on Rink amide-PS resin:

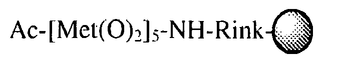

### Step 1: coupling of the first methionine sulfone residue on Rink amide-PS resin.

100 mg of Fmoc-Rink amide resin AM-PS 0.6 mmol/g was swelled for 10 minutes in dichloromethane and submitted to standard deprotection cycle (1.2 ml of DMF /piperidine 80/20 v/v solution for 20 minutes). The resin is then washed twice with dimethylformamide, twice with methanol, and then twice with dichloromethane. After washing step, the sulfone derivative of N-Fmoc protected methionine was loaded on the resin through a standard coupling cycle, using 40µl of 0.5 M solution of N-Fmoc protected methionine sulfone in dimethylformamide; 400µl of 0.5 M solution of N-methyl morpholine in dimethylformamide and 400µl of 0.5 M solution of HBTU in dimethylformamide. Coupling time was 90 min. Resin was washed as usual and N-Fmoc protecting group was removed by deprotected by a 1.2 ml of DMF / piperidine 80/20 v/v solution for 20 minutes.

### Step 2: stepwise elongation of the polymethionine sulfone polymer

The resin obtained in step 1 was submitted to four successive standard solid phase peptide synthesis cycles.

Each cycle is composed of a coupling step followed by a N-Fmoc deprotection step.
- Coupling step: Resin was added to 400µl of 0.5 M solution of N-Fmoc protected methionine sulfone in dimethylformamide; 400µl of 0.5 M solution of N-methyl morpholine in dimethylformamide and 400µl of 0.5 M solution of HBTU in dimethylformamide. Coupling time was 90 min. The resin is then washed twice with dimethylformamide, twice with methanol, and then twice with dichloromethane.
- Deprotection step was carried out using 1.2 ml of DMF / piperidine 80/20 v/v solution for 20 minutes. The resin is then washed as usual twice with dimethylformamide, twice with methanol, and then twice with dichloromethane.

### Step 3: Acetylation of the resin

The resin obtained in step 1, bearing methionine sulfone polymers, is acetylated for 5 minutes with a 50/50 acetic acid/dichloromethane solution (20 ml for 1.5 g of resin dried in a desiccator) in rotary solid-phase reactors, and then filtered. The acetylation is begun again for 10 minutes. The resin is then washed twice with dichloromethane, twice with methanol, and then twice with dichloromethane.

### Example 8: Cyclization of the CCAP peptide by formation of disulfide bridges with sulfone and sulfoxyde type supported reagent:

The formula of the CCAP peptide is as follows:
H-Pro-Phe-Cys-Asn-Ala-Phe-Thr-Gly-Cys-NH₂

Three experiments were carried out in buffer B of example 6 with 3 different oxidation reactants.
- DMSO in solution at 10% in the chosen buffer,
- Ac-Met(O) supported on NH₂-PL-PEGA
- Ac-Met(O)₂ supported on NH₂-PL-PEGA

The CCAP peptide is solubilized in buffer at a concentration of 3 mM and aliquots of 2 ml of the solution obtained are distributed into each of the 3 plastic syringes in parallel. The two supported oxidation reactants were used at 5 equivalents relative to the amount of peptide to be oxidized. The amount of supported reactant (5 eq) is calculated relative to the load of the resin.

Thus, 75 mg (5 eq) of Ac-Met(O) or Ac-Met(O)₂ supported on NH₂-PL-PEGA functionalized at 0.4 mmol/g were added to 2 ml of CCAP peptide solution. The three reactors were shaken on an orbital shaker (600 rpm) at ambient temperature for 96 hours and samples of 40 µl made up to 200 µl with a CH₃CN/H₂O (50/50) solution and then filtered were immediately subjected to LC/MS analyses. The LC/MS experiments were carried out on a quadripolar/time of flight mass spectrometer of Micromass Q-Tof type coupled to a Waters Alliance HPLC system. These analyses made it possible to quantify the presence of cyclized oxidized peptide as a function of time. The percentage of oxidized peptide given in table (III) was calculated as a function of the areas of the chromatographic peaks obtained at 214 nm. This value therefore takes into account the possible presence of by-products, but not the presence of DMSO, in experiment #1.

**Table (III) Percentage of oxidized peptide as a function of the oxidation reactant.**

| # **Exp.** | **Oxidant** | **30 sec** | **30 min** | **240 min** | **96 h** |
|---|---|---|---|---|---|
| 1 | DMSO 10% | | 14 | 30 | 100 |
| 2 | Met(O₂) | 5 | 26 | 62 | 100 |
| 3 | Met(O) | 5 | 20 | 55 | 100 |

Under the conditions tested, the supported reactant according to the invention bearing only one methionine sulfone derivative (experiment #2) proves to be more rapid than DMSO in solution and slighty more rapid than the methionine sulfoxyde counterparts. The chromatograms obtained confirm, in this case, the absence of these by-products, in particular the absence of covalent dimer derived from an intermolecular reaction between two CCAP peptides.

## Claims

1. Oxidation reactant of formula (I): 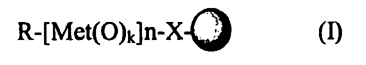 in which,
■ R represents a hydrogen atom or an amine function-protecting group, advantageously chosen from linear or branched (C₁-C₆) alkylcarbonyl, linear or branched (C₁-C₆) alkyloxycarbonyl, linear or branched (C₂-C₆) alkenyloxycarbonyl, linear or branched (C₂-C₆) alkynyloxycarbonyl, and aryloxycarbonyl groups, it being possible for each of these groups to be substituted with one or more halogen atoms or hydroxyl, amino, or linear or branched (C₁-C₆) mono- or dialkylamino groups, in particular chosen from a hydrogen atom and acyl, acetyl, benzyloxycarbonyl, tert-butyloxycarbonyl, and 9-fluorenylmethyloxycarbonyl groups.
■ Met represents a methionine residue of formula 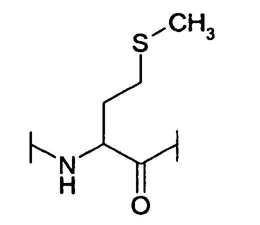
■ [Met(O)ₖ] represents an oxidized methionine residue of formula 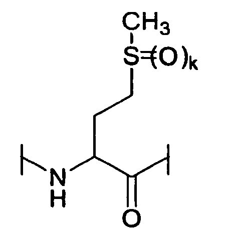
■ 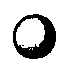 represents a solid or soluble support used in organic synthesis, advantageously chosen from gelatinous or macroporous supports having a matrix with a polystyrene, polyamide, polyethylene glycol or composite base, it being possible for said supports to be in the form of beads, of film-coated supports such as rings or lanterns, of plugs, or of noncrosslinked soluble supports, and it being possible for said matrix to be nonfunctionalized or functionalized with a functionalized arm, in particular a resin without a functionalized arm or a functionalized resin such as chloromethyl polystyrene, benzyloxybenzyl alcohol polystyrene, aminomethyl polystyrene, or methylbenzhydrylamine polystyrene resins, or resins grafted with a functionalized arm chosen from Rink amide, chlorotrityl, hydroxymethylbenzylacetamide, Sieber amide, aminomethyl-3,5-dimethoxyphenoxyalkyl, aminomethyl-3-dimethoxyphenoxyalkyl, hydroxymethyl-3,5-dimethoxyphenoxyalkyl, and hydroxymethyl-3-dimethoxyphenoxyalkyl.
■ X represents a radical derived from a function of the type amine or hydroxyl, or halogeno, capable of anchoring methionine, it being understood that this function capable of anchoring methionine can be separated from the support by means of a functionalized arm or a spacer,
■ n is between 2 and less than 10000, and
■ k is 1 or 2, or
■ n is equal to 1 and k is equal to 2.

2. Use of an oxidation reactant according to Claim 1, for the formation of intramolecular disulfide bridges, advantageously in peptides and/or proteins, in particular for the preparation of a library of peptides comprising at least one intramolecular disulfide bridge, by high-throughput parallel synthesis.

3. Process for preparing an oxidation reactant according to Claim 1, **characterized in that** the compound of formula (II) 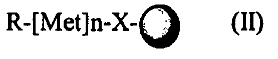 in which R, n, 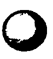 and X are as defined in Claim 1, is subjected to an oxidation reaction, to give the compound of formula (I): 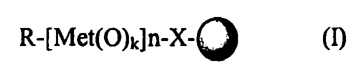 in which R, n, k and X are as defined in Claim 1.

4. Preparation process according to Claim 3, **characterized in that** the compound of formula (II) is prepared from the compound of formula (III):
R-[Met]-OH (III)
in which R is as defined in Claim 1,
by reaction with a support of formula 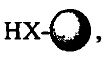 in which X and 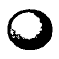 have the same meaning as in Claim 1,
to give the compound of formula (IV): 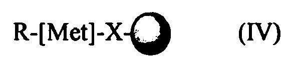 in which R, X, and 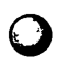 are as defined above,
which compound of formula (IV), after deprotection by cleavage of the R group, is subjected to a series of reactions with a compound of formula (III):
R-[Met]-OH (III)
in which R is as defined above,
the reaction being repeated n-1 times to give a compound of formula (II): 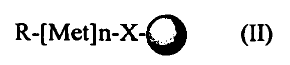 in which R, n, X, and 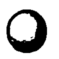 are as defined above,
it being understood that the protective group R can be removed and/or modified at any moment of the synthesis depending on the reactants used.

5. Preparation process according to Claim 3, **characterized in that** the compound of formula (II) is prepared from the N-carboxyanhydride of formula (V): 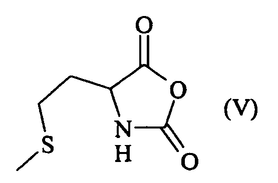 by polymerization in the presence of a support of formula 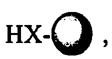 in which X and 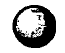 have the same meaning as in Claim 1, in a solvent such as THF, for a period of between 12 and 48 hours,
to give a compound of formula (II'): 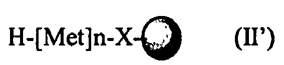 an analog of the compound of formula (II) in which R represents a hydrogen atom, it being possible for the latter to be replaced with a protective group, to give a compound of formula (II) as defined in Claim 3.

6. Process for preparing an oxidation reactant according to Claim 1, **characterized in that** the compound of formula (VI):
R-[Met(O)ₖ]-OH (VI)
in which R and k are as defined in Claim 1,
is condensed with a support of formula 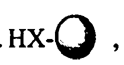 in which X and 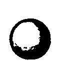 have the same meaning as in Claim 1,
to give a compound of formula (VII): 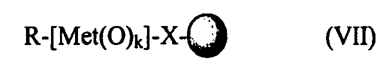 in which R, X, k and 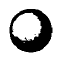 are as defined above,
which compound of formula (VII), after deprotection by cleavage of the R group, is subjected to a series of reactions with a compound of formula (VI):
R-[Met(O)]-OH (VI)
in which R is as defined above,
by means of a conventional peptide coupling method, in the presence of a coupling agent, the reaction being repeated n-1 times, to give a compound of formula (I) as defined in Claim 1.

7. Process for preparing an oxidation reactant according to Claim 1, **characterized in that** the compound of formula (IX): 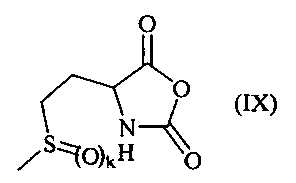 in which k is as defined in Claim 1, is polymerized in the presence of a support of
formula 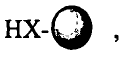 in which X and 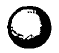 have the same meaning as in Claim 1, in a solvent such as THF, for a period of between 12 and 48 hours, to give a compound of formula (I'): 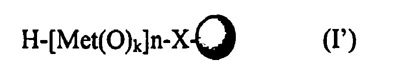 in which n, k, X, and 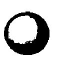 have the same meaning as above, the N-terminal portion of which compound (I') is protected with an R group, to give a compound of formula (I) as defined in Claim 1.

8. Process for preparing an oxidation reactant according to Claim 1, **characterized in that** the compound of formula (X) :
R-[Met(O)ₖ]n-OH (X)
in which R, k and n are as defined in Claim 1,
reacts with a support of formula 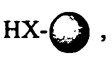 in which X and 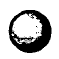 have the same meaning as in Claim 1,
to give the compound of formula (I) as defined in Claim 1.

9. Preparation process according to Claim 8, **characterized in that** the compound of formula (X) is prepared by means of a series of reactions consisting of condensation of the compound of formula (III'):
H-[Met]-OR' (III')
in which R' represents a linear or branched (C₁-C₆) alkyl, linear or branched (C₂-C₆) alkenyl, benzyl, carboxamidomethyl, or benzhydryl glycol amide ester group,
with a compound of formula (III):
R-[Met]-OH (III)
in which R is as defined above,
by means of a conventional peptide coupling method, in the presence of a coupling agent, the reaction being repeated n-1 times, to give, after protection of the C-terminal function, a compound of formula (XI):
R-[Met]n-OR' (XI)
in which R, R', and n are as defined above, it being understood that R is different from a hydrogen atom,
which compound of formula (XI) is subjected to an oxidation reaction, followed by a deprotection reaction by cleavage of the R' group, to give a compound of formula (X) as defined in Claim 8.

10. Preparation process according to Claim 9, **characterized in that** the compound of formula (XI) is prepared from the N-carboxyanhydride of formula (V): 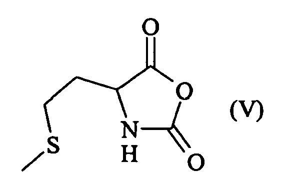 which compound of formula (V) is left in the open air without solvent for 2 days, to give, by means of a spontaneous polymerization reaction initiated by the moisture in the air, the compound of formula (XI'):
H-[Met]n-OH (XI')
an analog of the compound of formula (XI) for which R and R' each represent a hydrogen atom, it being possible for the latter to be replaced with a protective group, to give a compound of formula (XI) as defined in Claim 9.

11. Preparation process according to Claim 8, **characterized in that** the compound of formula (X) is prepared by polycondensation of the compound of formula (VI'):
H-[Met(O)ₖ]-OR' (VI')
in which R' represents a linear or branched (C₁-C₆) alkyl, linear or branched (C₂-C₆) alkenyl, benzyl, carboxamidomethyl, or benzhydryl glycol amide ester group, and k is as defined in Claim 1,
with a compound of formula (VI):
R-[Met(O)ₖ]-OH (VI)
in which R and k are as defined in Claim 1,
by means of a conventional peptide coupling method, in the presence of a coupling agent, the reaction being repeated n-1 times, to give a compound of formula (X), after deprotection by cleavage of the R' group.

12. Preparation process according to Claim 8, **characterized in that** the compound of formula (X) is prepared by polymerization of the N-carboxyanhydride of formula (IX): 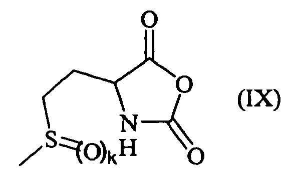 in which k is as defined above,
which is left in the open air without solvent for 2 days, the spontaneous polymerization reaction being initiated by the moisture in the air,
to give a compound of formula (X'):
H-[Met(O)ₖ]n-OH (X')
in which n and k are as defined above,
the N-terminal portion of which is protected with an R group, to give a compound of formula (X) as defined in Claim 3.

13. Oxidation reactant of formula 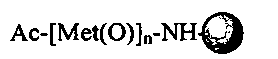 in which Ac represents acetyl, n is an integer selected from 1 to 9, 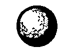 represents a rink amide-polystyrene resin or
a polyethylene glycol-polystyrene resin or
a polyamide-polyethylene glycol-dimethylacrylamide resin.

14. Oxidation reactant according to claim 1 of formula 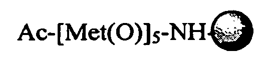 in which Ac represents acetyl and 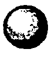 represents a polyethylene glycol-polystyrene resin.

15. Oxidation reactant according to claim 1 of formula 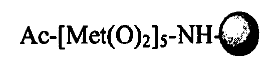 in which Ac represents acetyl and 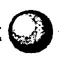 represents a rink amide-polystyrene resin.

16. Oxidation reactant according to claim 1 of formula 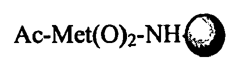 in which Ac represents acetyl and 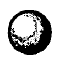 represents a polyamide-polyethylene glycol-dimethylacrylamide resin.

## Patentansprüche

1. Oxidationsreagenz der Formel (I): 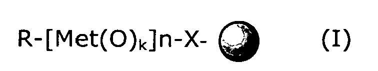 in welcher
- R ein Wasserstoffatom oder eine Schutzgruppe für eine Aminfunktion repräsentiert, vorteilhafterweise ausgewählt aus linearen oder verzweigten (C₁-C₆) Alkylcarbonylgruppen, linearen oder verzweigten (C₁-C₆) Alkyloxycarbonylgruppen, linearen oder verzweigten (C₂-C₆) Alkenyloxycarbonylgruppen, linearen oder ver-' zweigten (C₂-C₆) Alkinyloxycarbonylgruppen und Aryloxycarbonylgruppen, wobei jede dieser Gruppen substituiert sein kann mit einem oder mehreren Halogenatomen oder einer oder mehreren Hydroxylgruppen, Aminogruppen oder linearen oder verzweigten (C₁-C₆) Mono- oder Dialkylaminogruppen, insbesondere ausgewählt aus einem Wasserstoffatom und Acyl-, Acetyl-, Benzyloxycarbonyl-, tert-Butyloxycarbonyl- und 9-Fluorenylmethyloxycarbonylgruppen,
- Met einen Methioninrest repräsentiert der Formel 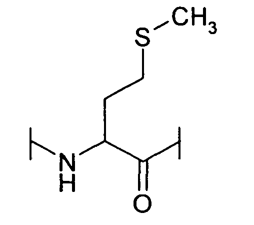
- [Met(O)ₖ] einen oxidierten Methioninrest repräsentiert der Formel 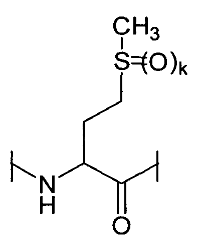
- 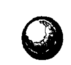 einen festen oder löslichen Träger, der in der organischen Synthese verwendet wird, repräsentiert, vorteilhafterweise ausgewählt aus gelartigen oder makroporösen Trägern mit einer Matrix mit einer Polystyrol-, Polyamid-, Polyethylenglykol- oder zusammengesetzten Basis, wobei diese Träger in Form von Kügelchen, von filmbeschichteten Trägern wie Ringen oder Laternen, von Pfropfen oder von nicht-vernetzten löslichen Trägern vorliegen können, und wobei die Matrix nicht-funktionalisiert sein kann oder funktionalisiert sein kann mit einem funktionalisierten Arm, insbesondere ein Harz ohne einen funktionalisierten Arm oder ein funktionalisiertes Harz wie Chloromethyl-Polystyrol-, Benzyloxybenzylalkohol-Polystyrol-, Aminomethyl-Polystyrol- oder Methylbenzhydrylamin-Polystyrolharze, oder Harze, die gepfropft sind mit einem funktionalisierten Arm ausgewählt aus Rink-Amid, Chlorotrityl, Hydroxymethylbenzylacetamid, Sieber-Amid, Aminomethyl-3,5-dimethoxyphenoxyalkyl, Aminomethyl-3-dimeth-oxyphenoxyalkyl, Hydroxymethyl-3,5-dimethoxyphenoxyalkyl und Hydroxymethyl-3-dimethoxyphenoxyalkyl,
- X ein Radikal repräsentiert, das abgeleitet ist von einer Funktion vom Typ Amin oder Hydroxyl oder Halogeno, welches in der Lage ist Methionin zu verankern, wobei es sich versteht, dass diese zur Verankerung von Methionin fähige Funktion von dem Träger mittels einem funktionalisierten Arm oder Spacer getrennt sein kann,
- n zwischen 2 und weniger als 10000 ist, und
- k 1 oder 2 ist, oder
- n gleich 1 und k gleich 2 ist.

2. Verwendung eines Oxidationsreagenz nach Anspruch 1 zur Bildung von intramolekularen Disulfidbrücken, vorteilhafterweise in Peptiden und/oder Proteinen, insbesondere für die Herstellung einer Bibliothek von Peptiden umfassend mindestens eine intramolekulare Disulfidbrücke mittels einer Parallelsynthese mit hohem Durchsatz.

3. Verfahren zur Herstellung eines Oxidationsreagenz nach Anspruch 1, **dadurch gekennzeichnet, dass** die Verbindung der Formel (II): 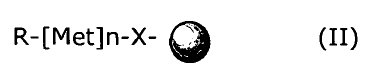 in welcher R, n, 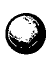 und X wie in Anspruch 1 definiert sind, einer Oxidationsreaktion unterworfen wird, um die Verbindung der Formel (I) zu ergeben: 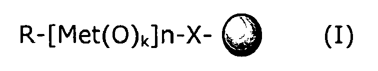 in welcher R, n, k und X wie in Anspruch 1 definiert sind.

4. Herstellungsverfahren nach Anspruch 3, **dadurch gekennzeichnet, dass** die Verbindung der Formel (II) aus der Verbindung der Formel (III) hergestellt wird:
R-[Met]-OH (III)
in welcher R wie in Anspruch 1 definiert ist,
durch Reaktion mit einem Träger der Formel 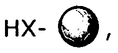 in welcher X und 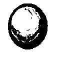 dieselbe Bedeutung haben wie in Anspruch 1, um die Verbindung der Formel (IV) zu ergeben: 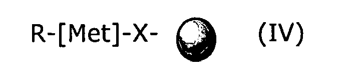 in welcher R, X und 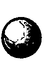 wie oben definiert sind,
wobei die Verbindung der Formel (IV) nach dem Entschützen durch Abspaltung der Gruppe R einer Serie von Reaktionen mit einer Verbindung der Formel (III) unterworfen wird:
R-[Met]-OH (III)
in welcher R wie oben definiert ist,
wobei die Reaktion n-1 mal wiederholt wird, um eine Verbindung der Formel (II) zu ergeben: 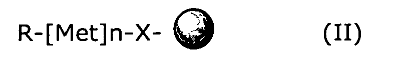 in welcher R, n, X und 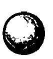 wie oben definiert sind,
wobei es sich versteht, dass die Schutzgruppe R in Abhängigkeit von den verwendeten Reaktanden zu jeder Zeit der Synthese entfernt und/oder modifiziert werden kann.

5. Herstellungsverfahren nach Anspruch 3, **dadurch gekennzeichnet, dass** die Verbindung der Formel (II) hergestellt wird aus dem N-Carboxyanhydrid der Formel (V): 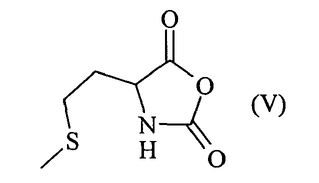 durch Polymerisation in Gegenwart eines Trägers der Formel 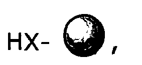 in welcher X und 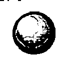 dieselbe Bedeutung haben wie in Anspruch 1, in einem Lösungsmittel wie THF für einen Zeitraum zwischen 12 und 48 Stunden,
um eine Verbindung der Formel (II') zu ergeben: 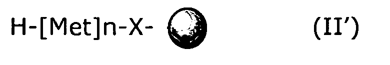 ein Analogon der Verbindung der Formel (II), in welcher R ein Wasserstoffatom repräsentiert, wobei letzteres durch eine Schutzgruppe ersetzt sein kann, um eine Verbindung der Formel (II), wie in Anspruch 3 definiert, zu ergeben.

6. Verfahren zur Herstellung eines Oxidationsreagens nach Anspruch 1, **dadurch gekennzeichnet, dass** die Verbindung der Formel (VI):
R-[Met(O)ₖ]-OH (VI)
in welcher R und k wie in Anspruch 1 definiert sind,
mit einem Träger der Formel 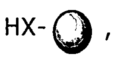 in welcher X und 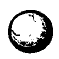 dieselbe Bedeutung haben wie in Anspruch 1, kondensiert wird, um eine Verbindung der Formel (VII) zu ergeben: 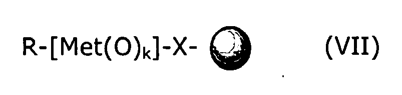 in welcher R, X, k und 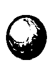 wie oben definiert sind, wobei die Verbindung der Formel (VII) nach dem Entschützen durch Abspaltung der Gruppe R einer Serie von Reaktionen mit einer Verbindung der Formel (VI) unterworfen wird:
R-[Met(O)]-OH (VI)
in welcher R wie oben definiert ist,
mittels einer konventionellen Peptid-Kopplungsmethode in Gegenwart eines Kopplungsagens, wobei die Reaktion n-1 mal wiederholt, um eine Verbindung der Formel (I), wie in Anspruch 1 definiert, zu ergeben.

7. Verfahren zur Herstellung eines Oxidationsreagens nach Anspruch 1, **dadurch gekennzeichnet, dass** die Verbindung der Formel (IX): 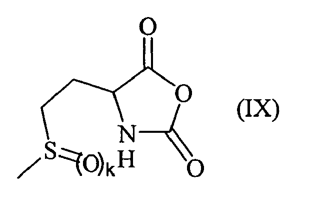 in welcher k wie in Anspruch 1 definiert ist, in Gegenwart eines Trägers
der Formel 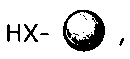 in welcher X und 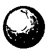 dieselbe Bedeutung haben wie in Anspruch 1, in einem Lösungsmittel wie THF für einen Zeitraum zwischen 12 und 48 Stunden polymerisiert wird,
um eine Verbindung der Formel (I') zu ergeben: 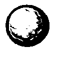 H-[Met(O)ₖ]n-X- (I')
in welcher n, k, X und 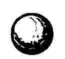 dieselbe Bedeutung haben wie oben, wobei der N-terminale Teil der Verbindung (I') mit einer Gruppe R geschützt wird, um eine Verbindung der Formel (I), wie in Anspruch 1 definiert, zu ergeben.

8. Verfahren zur Herstellung eines Oxidationsreagens nach Anspruch 1, **dadurch gekennzeichnet, dass** die Verbindung der Formel (X):
R-[Met(O)ₖ]n-OH (X)
in welcher R, k und n wie in Anspruch 1 definiert sind,
mit einem Träger der Formel 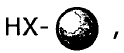 in welcher X und 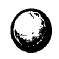 dieselbe Bedeutung haben wie in Anspruch 1, reagiert,
um eine Verbindung der Formel (I), wie in Anspruch 1 definiert, zu ergeben.

9. Herstellungsverfahren nach Anspruch 8, **dadurch gekennzeichnet, dass** die Verbindung der Formel (X) mittels einer Serie von Reaktionen hergestellt wird, die aus der Kondensation der Verbindung der Formel (III') bestehen:
H-[Met]-OR' (III')
in welcher R' eine lineare oder verzweigte (C₁-C₆) Alkylgruppe, eine lineare oder verzweigte (C₂-C₆) Alkenylgruppe, eine Benzyl-, Carboxamidomethyl- oder Benzhydryl-Glykolamid-Ester-Gruppe repräsentiert,
mit einer Verbindung der Formel (III):
R-[Met]-OH (III)
in welcher R wie oben definiert ist,
mittels einer konventionellen Peptid-Kopplungsmethode in Gegenwart eines Kopplungsagens, wobei die Reaktion n-1 mal wiederholt wird, um nach dem Schützen der C-terminalen Funktion eine Verbindung der Formel (XI) zu ergeben:
R-[Met]n-OR' (XI)
in welcher R, R' und n wie oben definiert sind, wobei es sich versteht, dass R von einem Wasserstoffatom verschieden ist, wobei die Verbindung der Formel (XI) einer Oxidationsreaktion unterworfen wird, gefolgt von einer Entschützungsreaktion durch Abspaltung der Gruppe R', um eine Verbindung der Formel (X), wie in Anspruch 8 definiert, zu ergeben.

10. Herstellungsverfahren nach Anspruch 9, **dadurch gekennzeichnet, dass** die Verbindung der Formel (XI) hergestellt wird aus dem N-Carboxyanhydrid der Formel (V): 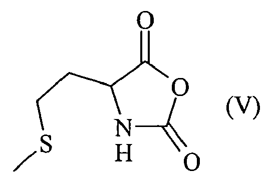 wobei die Verbindung der Formel (V) für zwei Tage ohne Lösungsmittel an der offenen Luft gelassen wird, um mittels einer spontanen Polymerisationsreaktion, die durch die Feuchtigkeit in der Luft initiiert wird, die Verbindung der Formel (XI') zu ergeben:
H-[Met]n-OH (XI')
ein Analogon der Verbindung der Formel (XI) bei der R und R' jeweils ein Wasserstoffatom repräsentieren, wobei letzteres durch eine Schutzgruppe ersetzt werden kann, um eine Verbindung der Formel (XI), wie in Anspruch 9 definiert, zu ergeben.

11. Herstellungsverfahren nach Anspruch 8, **dadurch gekennzeichnet, dass** die Verbindung der Formel (X) hergestellt wird durch Polykondensation der Verbindung der Formel (VI'):
H-[Met(O)ₖ]-OR' (VI')
in welcher R' eine lineare oder verzweigte (C₁-C₆) Alkylgruppe, eine lineare oder verzweigte (C₂-C₆) Alkenylgruppe, eine Benzyl-Carboxamidomethyl- oder Benzhydryl-Glykolamid-Ester-Gruppe repräsentiert, und k wie in Anspruch 1 definiert ist,
mit einer Verbindung der Formel (VI):
R-[Met(O)ₖ]-OH (VI)
in welcher R und k wie in Anspruch 1 definiert sind,
mittels einer konventionellen Peptid-Kopplungsmethode in Gegenwart eines Kopplungsagens, wobei die Reaktion n-1 mal wiederholt wird, um nach dem Entschützen durch Abspaltung der Gruppe R' eine Verbindung der Formel (X) zu ergeben.

12. Herstellungsverfahren nach Anspruch 8, **dadurch gekennzeichnet, dass** die Verbindung der Formel (X) hergestellt wird durch Polymerisation des N-Carboxyanhydrids der Formel (IX): 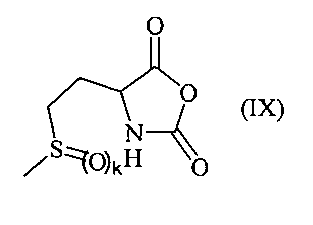 in welcher k wie oben definiert ist,
welche für zwei Tage ohne Lösungsmittel an der offenen Luft gelassen wird, wobei die spontane Polymerisationsreaktion durch die Feuchtigkeit in der Luft initiiert wird,
um eine Verbindung der Formel (X') zu ergeben:
H-[Met(O)ₖ]n-OH (X')
in welcher n und k wie oben definiert sind,
wobei deren N-terminaler Teil mit einer Gruppe R geschützt wird, um eine Verbindung der Formel (X), wie in Anspruch 3 definiert, zu ergeben.

13. Oxidationsreagenz der Formel 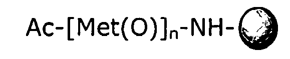 in welcher Ac Acetyl repräsentiert, n eine ganze Zahl ist ausgewählt von 1 bis 9, 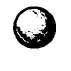 ein Rink-Amid-Polystyrolharz oder
ein Polyethylenglykol-Polystyrolharz oder
ein Polyamid-Polyethylenglykol-Dimethylacrylamidharz repräsentiert.

14. Oxidationsreagenz nach Anspruch 1 der Formel 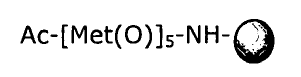 in welcher Ac Acetyl repräsentiert und 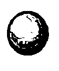 ein Polyethylenglykol-Polystyrolharz repräsentiert.

15. Oxidationsreagenz nach Anspruch 1 der Formel 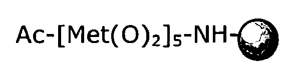 in welcher Ac Acetyl repräsentiert und 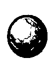 ein Rink-Amid-Polystyrolharz repräsentiert.

16. Oxidationsreagenz nach Anspruch 1 der Formel 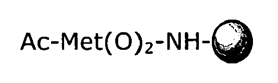 in welcher Ac Acetyl repräsentiert und 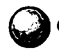 ein Polyamid-Polyethylenglykol-Dimethylacrylamidharz repräsentiert.

## Revendications

1. Réactif d'oxydation de formule (I) : 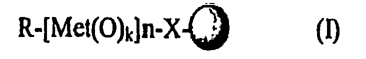 dans lequel :
- R représente un atome d'hydrogène ou un groupe de protection de fonction amine, qui est avantageusement choisi parmi des groupes (C₁-C₆) alkylcarbonyle linéaires ou ramifiés, (C₁-C₆) alkyloxycarbonyle linéaires ou ramifiés, (C₂-C₆) alcényloxycarbonyle linéaires ou ramifiés, (C₂-C₆) alcynylocarboxyle linéaires ou ramifiés, et aryloxycarbonyle, chacun de ces groupes pouvant être substitué avec un ou plusieurs atome(s) d'halogène ou groupe(s) hydroxyle, amino ou (C₁-C₆) mono- ou dialkylamino linéaire(s) ou ramifié(s), en particulier choisis parmi un atome d'hydrogène et des groupes acyle, acétyle, benzyloxycarbonyle, tert-butyloxycarbonyle et 9-fluorénylméthyloxycarbonyle ;
- Met représente un résidu de méthionine de formule : 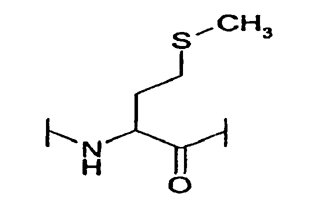
- [Met(O)ₖ] représente un résidu de méthionine oxydé de formule : 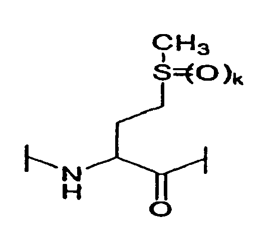 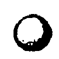 représente un support solide ou soluble utilisé lors d'une synthèse organique, qui est avantageusement choisi parmi des supports gélatineux ou macro-poreux comprenant une matrice possédant une base de polystyrène, de polyamide, de polyéthylène glycol ou composite, chacun desdits supports pouvant se présenter sous la forme de perles, de supports revêtus d'un film tels que des anneaux ou des lanternes, de bouchons ou de supports solubles non réticulés, et ladite matrice pouvant être non fonctionnalisée ou fonctionnalisée avec une branche fonctionnalisée, en particulier une résine sans branche fonctionnalisée ou une résine fonctionnalisée telle des résines de chlorométhyl polystyrène, de benzyloxybenzyl alcool polystyrène, d'aminométhyl polystyrène ou de méthylbenzhydrylamine polystyrène, ou des résines greffées avec une branche fonctionnalisée choisie parmi les résines Rink amide, chlorotrityl, hydroxyméthyl-benzylacétamide, Sieber amide, aminométhyl-3,5-diméthoxyphénoxyalkyle, aminométhyl-3-diméthoxyphénoxyalkyle, hydroxyméthyl-3,5-diméthoxyphénoxyalkyle et hydroxyméthyl-3-diméthoxyphénoxyalkyle ;
- X représente un radical dérivé d'une fonction du type amine ou hydroxyle, ou halogène, capable d'ancrer la méthionine, étant entendu que cette fonction capable d'ancrer la méthionine peut être séparée du support au moyen d'une branche fonctionnalisée ou d'un espaceur ;
- n est compris entre 2 et moins de 10000 ; et
- k est égal à 1 ou 2 ; et
- n est égal à 1 et k est égal à 2.

2. Utilisation d'un réactif d'oxydation selon la revendication 1, pour la formation de liaisons disulfures intra-moléculaires, avantageusement dans des peptides et/ou dans des protéines, en particulier pour la préparation d'une bibliothèque de peptides comprenant au moins une liaison disulfure intramoléculaire, par une synthèse parallèle à haut débit.

3. Procédé pour préparer un réactif d'oxydation selon la revendication 1, **caractérisé en ce que** le composé de formule (II) : 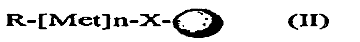 dans laquelle R, n , 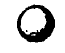 et X sont tels que définis dans la revendication 1, est soumis à une réaction d'oxydation, afin d'obtenir le composé de formule (I) : 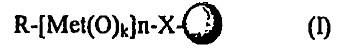 dans laquelle R, n, k et X sont tels que définis dans la revendication 1.

4. Procédé de préparation selon la revendication 3, **caractérisé en ce que** le composé de formule (II) est préparé à partir du composé de formule (III) :
**R-[Met]-OH** **(III)**
dans laquelle R est défini selon la revendication 1, par une réaction avec un support de formule 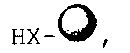 dans laquelle X et 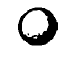 ont la même signification que dans la revendication 1, afin d'obtenir le composé de formule (IV) : 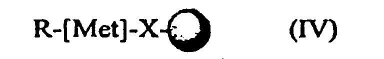 dans laquelle R, X et 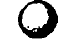 sont tels que définis ci-dessus, ledit composé de formule (IV), après déprotection par le clivage du groupe R, est soumis à une série de réactions avec un composé de formule (III) :
**R-[Met]-OH** **(III)**
dans laquelle R est tel qu'il est défini ci-dessus, la réaction étant répétée n-1 fois afin d'obtenir un composé de formule (II) : 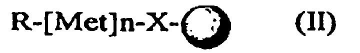 dans laquelle R, n, X et 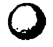 sont tels que définis ci-dessus, étant entendu que le groupe protecteur R peut être retiré et/ou modifié à n'importe quel moment de la synthèse en fonction des réactifs utilisés.

5. Procédé de préparation selon la revendication 3, **caractérisé en ce que** le composé de formule (II) est préparé à partir du N-carboxyanhydride de formule (V) : 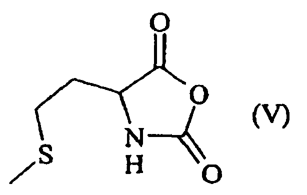 par polymérisation en présence d'un support de formule 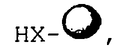 dans laquelle X et 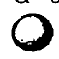 ont la même signification que dans la revendication 1, dans un solvant tel que le THF, pendant une période comprise entre 12 heures et 48 heures, afin d'obtenir un composé de formule (II') : 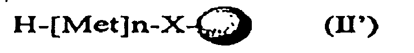 un composé analogue au composé de formule (II), dans laquelle R représente un atome d'hydrogène, ce dernier pouvant être remplacé par un groupe protecteur, afin d'obtenir un composé de formule (II) selon la revendication 3.

6. Procédé pour préparer un réactif d'oxydation selon la revendication 1, **caractérisé en ce que** le composé de formule (VI) :
**R-[Met(O)ₖ]-OH** **(VI)**
dans laquelle R et k sont tels qu'ils sont définis dans la revendication 1, est condensé avec un support de formule 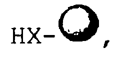 dans laquelle X et 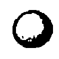 ont la même signification que dans la revendication 1, afin d'obtenir le composé de formule (VII) : 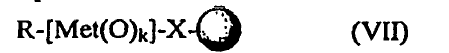 dans laquelle R, X, k et 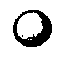 sont tels que définis ci-dessus, ledit composé de formule (VII), après déprotection par le clivage du groupe R, est soumis à une série de réactions avec un composé de formule (VI) :
**R-[Met(O)ₖ]-OH** **(VI)**
dans laquelle R est tel qu'il est défini ci-dessus, au moyen d'un procédé conventionnel de couplage peptidique, en présence d'un agent de couplage, la réaction étant répétée n-1 fois, afin d'obtenir un composé de formule (I) selon la revendication 1.

7. Procédé pour préparer un réactif d'oxydation selon la revendication 1, **caractérisé en ce que** le composé de formule (IX) : 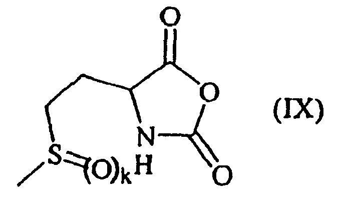 dans laquelle k est tel qu'il est défini dans la revendication 1, est polymérisé en présence d'un support de formule 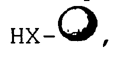 dans laquelle X et 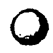 ont la même signification que dans la revendication 1, dans un solvant tel que le THF, pendant une période comprise entre 12 heures et 48 heures, afin d'obtenir un composé de formule (I') : 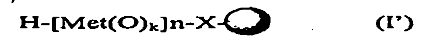 dans laquelle n, k, X et 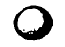 ont la même signification que ci-dessus, où la partie N-terminale du composé (I') est protégée par un groupe R, afin d'obtenir un composé de formule (I) selon la revendication 1.

8. Procédé pour préparer un réactif d'oxydation selon la revendication 1, **caractérisé en ce que** le composé de formule (X) :
**R-[Met(O)ₖ]n-OH** **(X)**
dans laquelle R, k et n sont tels que définis dans la revendication 1, réagit avec un support de formule 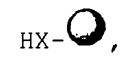 dans laquelle X et 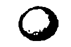 ont la même signification que dans la revendication 1, afin d'obtenir le composé de formule (I) selon la revendication 1.

9. Procédé de préparation selon la revendication 8, **caractérisé en ce que** le composé de formule (X) est préparé au moyen d'une série de réactions consistant en la condensation du composé de formule (III') :
**H-[Met]-OR'** **(III')**
dans laquelle R' représente un groupe (C₁-C₆) alkyle linéaire ou ramifié, (C₂-C₆) alcényle linéaire ou ramifié, benzyle, carboxamidométhyle ou benzhydryl glycol amide ester, avec un composé de formule (III) :
**R-[Met]-OH** **(III)**
dans laquelle R est tel que défini ci-dessus, au moyen d'un procédé conventionnel de couplage de peptidique, en présence d'un agent de couplage, la réaction étant répétée n-1 fois, afin d'obtenir, après la protection de la fonction C-terminale, un composé de formule (XI) :
**R-[Met]n-OR'** **(XI)**
dans laquelle R, R' et n sont tels que définis ci-dessus, étant entendu que R est différent d'un atome d'hydrogène, où ledit composé de formule (IX) est soumis à une réaction d'oxydation, suivie par une réaction de déprotection par le clivage du groupe R', afin d'obtenir un composé de formule (X) tel que défini dans la revendication 8.

10. Procédé de préparation selon la revendication 9, **caractérisé en ce que** le composé de formule (XI) est préparé à partir du N-carboxyanhydride de formule (V) : 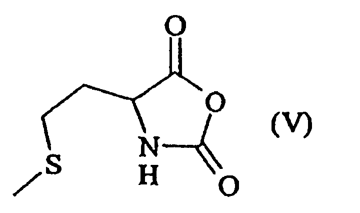 ledit composé de formule (V) est laissé à l'air libre sans solvant pendant 2 jours, afin d'obtenir, au moyen d'une réaction de polymérisation spontanée amorcée par l'humidité présente dans l'air, le composé de formule (XI') :
**H-[Met]n-OH** **(XI')**
un composé analogue du composé de formule (XI), dans laquelle R et R' représentent chacun un atome d'hydrogène, ce dernier pouvant être remplacé par un groupe protecteur, afin d'obtenir un composé de formule (XI) tel que défini dans la revendication 9.

11. Procédé de préparation selon la revendication 8, **caractérisé en ce que** le composé de formule (X) est préparé par une polycondensation du composé de formule (VI') :
**H-[Met(O)ₖ]-OR'** **(VI')**
dans laquelle R' représente un groupe (C₁-C₆) alkyle linéaire ou ramifié, (C₂-C₆) alcényle linéaire ou ramifié, benzyle, carboxamidométhyle ou benzhydryl glycol amide ester, et k est tel que défini dans la revendication 1, avec un composé de formule (VI) :
**R-[Met(O)ₖ]-OH** **(VI)**
dans laquelle R et k sont tels que définis dans la revendication 1, au moyen d'un procédé conventionnel de couplage de peptides, en présence d'un agent de couplage, la réaction étant répétée n-1 fois, afin d'obtenir un composé de formule (X), après la déprotection par le clivage du groupe R'.

12. Procédé de préparation selon la revendication 8, **caractérisé en ce que** le composé de formule (X) est préparé par une polymérisation du N-carboxyanhydride de formule (IX) : 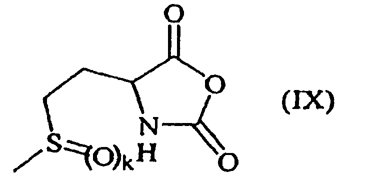 dans laquelle k est tel que défini ci-dessus, qui est laissé à l'air libre sans solvant pendant 2 jours, la réaction de polymérisation spontanée étant amorcée par l'humidité présente dans l'air, afin d'obtenir un composé de formule (X') :
**H-[Met(C»ₖ]n-OH** **(X')**
dans laquelle n et k sont tels que définis ci-dessus, dont la partie N-terminale est protégée avec un groupe R, afin d'obtenir un composé de formule (X) selon la revendication 3.

13. Réactif d'oxydation de formule : 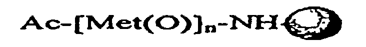 dans laquelle :
- Ac représente un acétyle ;
- n est un nombre entier sélectionné de 1 à 9 ;
et 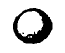 représente une résine Rink amide - polystyrène, ou une résine polyéthylène glycolpolystyrène, ou une résine polyamide - polyéthylène glycol - diméthylacrylamide.

14. Réactif d'oxydation selon la revendication 1, de formule : 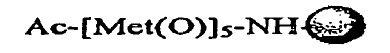 dans laquelle :
- Ac représente un acétyle ; et 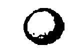 représente une résine polyéthylène glycol - polystyrène.

15. Réactif d'oxydation selon la revendication 1, de formule : 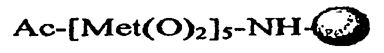 dans laquelle :
- Ac représente un acétyle ; et 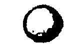 représente une résine Rink amide - polystyrène.

16. Réactif d'oxydation selon la revendication 1, de formule : 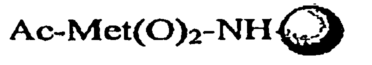 dans laquelle :
- Ac représente un acétyle ; et 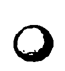 représente une résine polyamide - polyéthylène glycol - diméthylacrylamide.
